# FASCICULE DE BREVET EUROPEEN

(11) **EP 2 240 501 B1**
(45) Date de publication et mention de la délivrance du brevet: **14.10.2015**
(21) Numéro de dépôt: 09700113.5
(22) Date de dépôt: 05.01.2009
(51) Int. Cl.: C07F 9/38, A61K 31/663, A61P 35/00, A61P 19/10, A61P 3/14

(54) **DERIVES D'ACIDE HYDROXY-BISPHOSPHONIQUE COMME VECTEUR CIBLANT LE TISSU OSSEUX**
HYDROXY-BISPHOSPHONSÄUREDERIVATE ALS AUF KNOCHENGEWEBE GERICHTETE VEKTOREN
HYDROXY-BISPHOSPHONIC ACID DERIVATIVES AS VECTOR FOR TARGETING BONE TISSUE

(30) Priorité: 03.01.2008 FR 0850021
(43) Date de publication de la demande: 20.10.2010
(73) Titulaire: Université de Nantes, 44000 Nantes (FR); CHU Nantes, 44000 Nantes (FR); INSTITUT NATIONAL DE LA SANTE ET DE LA RECHERCHE MEDICALE (INSERM), 75013 Paris (FR); Centre National de la Recherche Scientifique (CNRS), 75016 Paris (FR)
(72) Inventeur: EGOROV, Maxim, F-44100 Nantes (FR); FORTUN, Yannick, F-44470 Mauves Sur Loire (FR); HEYMANN, Dominique, F-44610 Indre (FR); LEBRETON, Jacques, F-44300 Nantes (FR); MATHE, Monique, F-44300 Nantes (FR); PADRINES, Marc, F-44470 Carquefou (FR); REDINI, Françoise, F-44880 Sautron (FR)
(74) Mandataire: Regimbeau
(86) Numéro de dépôt international: PCT/EP2009/050027
(87) Numéro de publication internationale: WO 2009/083614

(56) Documents cités:
- EP-A- 0 387 194
- WO-A-97/12619
- WO-A-02/062398
- DE-A1- 4 011 777
- US-A- 5 110 807
- US-A1- 2004 214 798

## Description

La présente invention concerne de nouveaux dérivés d'acide hydroxy-bisphosphonique et leur utilisation en tant que vecteur ciblant le tissu osseux.

Le tissu osseux est un tissu conjonctif composé d'une fraction minérale constituée de phosphate de calcium sous forme de cristaux d'hydroxyapatite (Ca₁₀(PO₄)₆(OH)₂) et d'une fraction organique contenant une matrice extracellulaire et des cellules spécialisées.

Le tissu osseux est en perpétuel remaniement grâce à un processus appelé remodelage osseux. Il se caractérise par une phase d'apposition due à l'activité des ostéoblastes qui synthétisent une nouvelle matrice organique et induisent sa minéralisation (Owen et al., Curr. Opin. Nephrol. Hypertens. 1998, 7, 363) et une phase de dégradation assurée par les ostéoclastes qui résorbent la matrice organique et dissolvent le minéral (Roodman et al., Endocr. Rev. 1996, 17, 308). Ce processus physiologique permet de maintenir l'homéostasie phosphocalcique et la masse osseuse (Manologas et al., Endocriv. Rev. 2000, 21, 115) et de s'adapter aux contraintes mécaniques. Tout dérèglement de cet équilibre est lié à l'apparition de pathologies ostéocondensantes, telles que l'ostéopétrose, ou, le plus souvent, ostéolytiques pouvant être d'origine tumorale (avec des tumeurs primaires, comme l'ostéosarcome, ou secondaires, comme les métastases osseuses) ou non dans le cas de pathologies métaboliques comme l'ostéoporose.

Les bisphosphonates (forme basique de dérivés d'acide bisphosphonique dont font partie les dérivés d'acide hydroxy-bisphosphonique) sont des analogues synthétiques des pyrophosphates endogènes pour lesquels la chaîne P-O-P a été remplacée par une chaîne P-C-P, conduisant à des composés métaboliquement stables qui représentent un outil thérapeutique efficace des ostéolyses (Heymann et al., Trends Mol. Med., 2004, 10, 337).

Ces molécules ont tout d'abord été utilisées pour leur capacité à cibler le tissu osseux. De la même manière que les pyrophosphates, les bisphosphonates ont une forte affinité pour la partie minérale de l'os (affinité entre les groupements phosphates et le calcium de la partie minérale de l'os) et peuvent moduler à forte dose le processus de calcification. L'intérêt de telles substances a été mis en évidence pour le traitement de divers disfonctionnements du métabolisme osseux. Les bisphosphonates sont utilisés en particulier pour traiter les pathologies impliquant une résorption osseuse excessive conduisant d'une part à une hypercalcémie et d'autre part à des atteintes osseuses à l'origine de douleurs et de fractures. Ainsi, leur utilisation s'est imposée depuis une dizaine d'années pour le traitement de l'ostéoporose, de l'hypercalcémie d'origine tumorale ou non, ainsi que pour des pathologies ostéolytiques tumorales telles que le myélome multiple ou les métastases osseuses secondaires d'un carcinome prostatique ou mammaire. EP 0387194, DE 4011777,US 5110807, décrivent des biphosphonates utiles pour le traitement de pathologies du remodelage osseux.

Les études de structure-activité, développées à ce jour, ont clairement montré que la capacité des bisphosphonates à inhiber la résorption osseuse dépendait de deux facteurs structuraux :
- les groupes phosphonates (et hydroxyle dans le cas d'hydroxy-bisphosphonates), essentiels pour une bonne affinité du composé avec la partie minérale de l'os,
- la chaîne latérale R, spécifique d'une cible moléculaire, qui détermine l'activité biologique associée à la molécule.

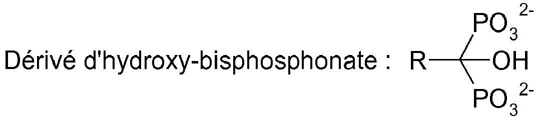

La présente invention concerne donc de nouveaux dérivés d'acide hydroxy-bisphosphonique répondant à la formule générale (I) suivante : 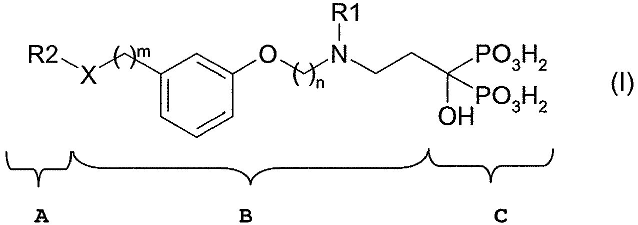 dans laquelle :
- n désigne 3,
- m désigne 1,
- X désigne un atome d'oxygène,
- R1 désigne un groupement méthyle et
- R2 désigne un résidu d'une molécule d'intérêt thérapeutique choisi parmi 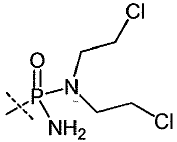 et 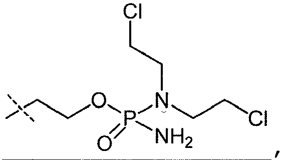
ou des sels pharmaceutiquement acceptables de ceux-ci.

Les molécules de l'invention sont donc composées de trois parties distinctes :
▪ une partie C correspondant à la fonction acide hydroxy-bisphosphonique qui va permettre à la molécule de cibler le tissu osseux de part la forte affinité de cette fonction pour la partie minérale de l'os,
▪ une partie B qui est un bras espaceur, sur lequel pourra être greffé le résidu R2, et enfin
▪ une partie A correspondant au résidu R2 d'une molécule à activité thérapeutique, permettant notamment un traitement ciblé d'une pathologie du tissu osseux.

Par groupement « alkyle », on entend une chaîne hydrocarbonée saturée, linéaire ou ramifiée, comportant de 1 à 6 atomes de carbone, comme par exemple un groupement, méthyle, éthyle, isopropyle, *tertio*-butyle, pentyle, etc.

Par « atome d'halogène », on entend un atome de fluor, de brome, de chlore ou d'iode.

Par « sel pharmaceutiquement acceptable », on entend notamment un sel obtenu à partir d'acides ou de bases pharmaceutiquement acceptables.

Parmi les acides pharmaceutiquement acceptables, on peut citer, à titre non limitatif, des acides inorganiques, tels que les acides halohydriques, comme les acides chlorhydrique et bromhydrique, les acides sulfurique, nitrique ou encore phosphorique, ou des acides organiques, tels que les acides acétique, propionique, benzoïque, lactique, pyruvique, oxalique, malonique, succinique, maléique, fumarique, malique, tartrique, citrique, mandélique, méthanesulfonique, p-toluènesulfonique, cyclamique, salicylique, aspartique, stéarique ou encore palmitique.

Parmi les bases pharmaceutiquement acceptables, on peut citer, à titre non limitatif, des bases inorganiques formant, par exemple, des sels d'ammonium ou des sels de métaux alcalins ou alcalino-terreux tels que le lithium, le sodium, le potassium, le magnésium ou encore le calcium, ou des bases organiques telles que la triéthylamine, la diisopropylamine, la pipéridine ou encore la morpholine.

Les dérivés d'acide hydroxy-bisphosphonique de formule (I) selon l'invention, destinés à une utilisation thérapeutique, sont les suivants :
▪ 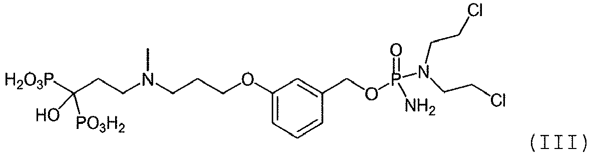 (molécule anti-tumorale potentiellement alkylante : applications envisagées à l'hypercalcémie maligne, aux tumeurs osseuses primitives et aux métastases osseuses), et
▪ 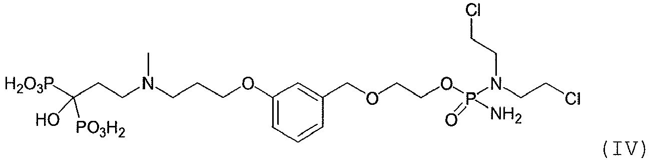 (molécule anti-tumorale potentiellement alkylante : applications envisagées à l'hypercalcémie maligne, aux tumeurs osseuses primitives et aux métastases osseuses).

La présente invention concerne également un dérivé d'acide hydroxy-bisphosphonique tel que décrit ci-dessus ou d'un sel pharmaceutiquement acceptable de celui-ci, pour son utilisation comme médicament, destiné notamment à cibler le tissu osseux.

En particulier, les dérivés d'acide hydroxy-bisphosphonique de l'invention pourront être utilisés pour le traitement d'une pathologie du remodelage osseux ostéolytique ou ostéocondensante, telle que les tumeurs osseuses primitives (telles qu'un ostéosarcome, un chondrosarcome, une tumeur à cellules géantes ou un sarcome d'Ewing), les métastases osseuses, le myélome multiple.

Selon un mode de réalisation, les dérivés d'acide hydroxy-bisphosphonique seront utilisés dans un traitement antitumoral, notamment pour traiter l'hypercalcémie maligne, les tumeurs osseuses primitives et les métastases osseuses.

La présente invention concerne par ailleurs une composition pharmaceutique comprenant au moins un dérivé d'acide hydroxy-bisphosphonique de l'invention, tel que décrit précédemment, ou un sel pharmaceutiquement acceptable de celui-ci et au moins un véhicule pharmaceutiquement acceptable.
Cette composition pourra être formulée de manière à permettre son administration, notamment, par voie sous-cutanée, intra-veineuse, per os, intramusculaire ou transdermique, c'est-à-dire de préférence sous forme d'une solution injectable ou sous forme d'un patch.

La présente invention a également pour objet une composition pharmaceutique telle que définie ci-dessus pour son utilisation en tant que médicament.

En particulier, les compositions de l'invention pourront être utilisés pour le traitement d'une pathologie du remodelage osseux ostéolytique ou ostéocondensante, telle que les tumeurs osseuses primitives, telles qu'un ostéosarcome, un chondrosarcome, une tumeur à cellules géantes ou un sarcome d'Ewing, les métastases osseuses, le myélome multiple.

Selon un mode de réalisation, les compositions thérapeutiques seront utilisées dans un traitement antitumoral, notamment pour traiter l'hypercalcémie maligne, les tumeurs osseuses primitives et les métastases osseuses.

Les composés de l'invention peuvent être préparés selon les étapes successives suivantes :
(a) couplage entre une composé de formule (A) suivante : 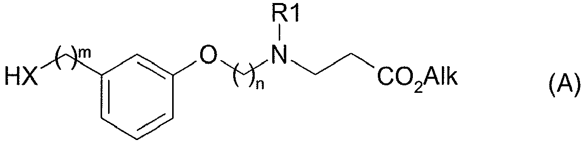 pour laquelle R1, X, n et m sont tels que définis ci-dessus et Alk représente un groupement alkyle linéaire ou ramifié en C₁ à C₆, et en particulier un groupe *tert*-butyle, et la molécule d'intérêt thérapeutique correspondant au résidu R2, pour donner un composé de formule (B) suivante : 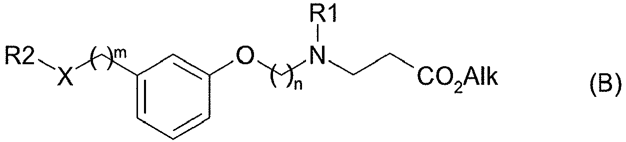 pour laquelle R1, R2, X, n, m et Alk sont tels que définis ci-dessus,
(b) saponification de l'ester terminal du composé de formule (B) obtenu à l'étape (a) précédente pour donner un composé de formule (C) suivante : 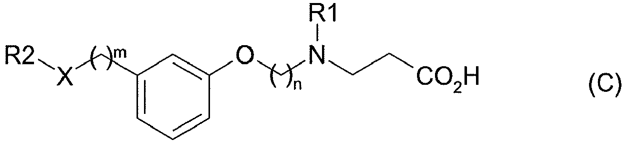 pour laquelle R1, R2, X, n et m sont tels que définis ci-dessus,
(c) conversion de la fonction acide carboxylique libre du composé de formule (C) obtenu à l'étape précédente en fonction hydroxy-bisphosphonique pour donner le composé de formule (I),
(d) éventuellement salification du composé de formule (I) obtenu à l'étape (c) précédente pour donner un sel pharmaceutiquement acceptable de celui-ci, et
(e) séparation du milieu réactionnel du composé de formule (I) ou d'un de ses sels pharmaceutiquement acceptables obtenu à l'étape (c) ou (d) précédente.

### Etape (a) :

Cette étape de couplage sera réalisée entre la fonction XH (c'est-à-dire OH ou NHR3) du composé de formule (A) et le groupement fonctionnel de la molécule d'intérêt thérapeutique telle que définie précédemment, par des techniques bien connues de l'homme du métier.

Il pourra s'agir en particulier d'une substitution nucléophile, comme notamment entre une molécule d'intérêt thérapeutique halogénée, notamment chlorée ou bromée, et la fonction XH du composé de formule (A). Une telle réaction pourra avantageusement être réalisée en présence d'une base telle que NaHCO₃ ou encore NaHMDS (hexaméthyldisilazane de sodium).

Il pourra s'agir également d'un couplage peptidique entre une fonction acide carboxylique portée par la molécule d'intérêt thérapeutique et la fonction XH portée par le composé de formule (A). Une telle réaction pourra être avantageusement réalisée en présence d'un agent de couplage éventuellement associé à un auxiliaire de couplage.

L'agent de couplage pourra être en particulier le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-H-benzotriazole-1-yl)-1,1,3,3-tetramethyluronium hexafluoro-phosphate (HBTU), le 2-(1H-benzotriazole-1-yl)-1,1,3,3-tetramethyluronium tetrafluoroborate (TBTU) ou le O-(7-azobenzotriazol-1-yl)-1,1,3,3-tétraméthyluronium hexafluoro-phosphate (HATU).

L'auxiliaire de couplage pourra être choisi notamment parmi le N-hydroxy succinimide (NHS), le N-hydroxy benzotriazole (HOBt), le 3,4-dihydro-3-hydroxy-4-oxo-1,2,3-benzotriazole (HOOBt), le 1-hydroxy-7-azabenzotriazole (HOAt), la diméthylaminopyridine (DMAP) ou le N-hydroxysylfosuccinimide (sulfo NHS).

### Etape (b) :

Cette étape pourra être réalisée en présence d'un acide tel que l'acide trifluoroacétique, formique, acétique, chlorhydrique, sulfurique, etc., et en particulier avec l'acide trifluoroacétique.

### Etape (c) :

Cette étape pourra être réalisée par des techniques bien connues de l'homme du métier.

Elle pourra être réalisée notamment par activation de la fonction acide carboxylique du composé (C) sous la forme de son dérivé boronate par action d'un borane tel que le catecholborane, puis réaction dans les conditions d'Arbuzov avec le tris(triméthylsilyl) phosphite, suivie d'un traitement avec un alcool aliphatique tel que le méthanol.

Par « alcool aliphatique », on entend un composé comportant une fonction alcool OH sur une chaîne hydrocarbonée linéaire ou ramifiée et saturée ou insaturée, et comportant avantageusement de 1 à 6, de préférence de 1 à 4, atomes de carbone.

### Etape (d) :

L'étape de salification sera réalisée en présence d'un acide ou d'une base pharmaceutiquement acceptable tel que défini ci-dessus.

### Etape (e) :

Le composé de formule (I) ainsi obtenu pourra être séparé du milieu réactionnel par des méthodes bien connues de l'homme du métier, comme par exemple par extraction, évaporation du solvant ou encore par précipitation et filtration.

Par ailleurs, ce composé pourra être purifié si nécessaire par des techniques bien connues de l'homme du métier, comme par recristallisation si le composé est cristallin, par chromatographie sur colonne sur gel de silice ou encore par chromatographie liquide haute performance (HPLC).

Le composé de formule (A), utilisé comme réactif de départ, pourra être préparé par des techniques bien connues de l'homme du métier.

Il pourra être préparé en particulier selon les étapes successives suivantes :
(a1) réaction entre un composé de formule (D) suivante : 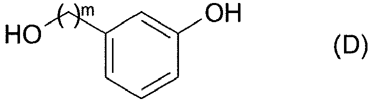 pour laquelle m est tel que défini ci-dessus,
   et une molécule de formule (E) suivante : 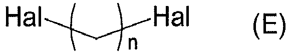 pour laquelle n est tel que défini ci-dessus et Hal représente un atome d'halogène, et en particulier un atome de brome,
   pour donner un composé de formule (F) suivante : 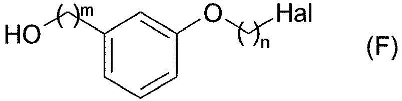 pour laquelle n, m et Hal sont tels que définis ci-dessus,
(b1) réaction entre le composé de formule (F) obtenu à l'étape (a1) précédente et un composé de formule (G) suivante : 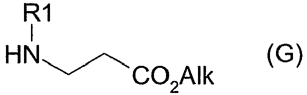 pour laquelle R1 et Alk sont tels que définis ci-dessus, pour donner un composé de formule (A1) suivante : 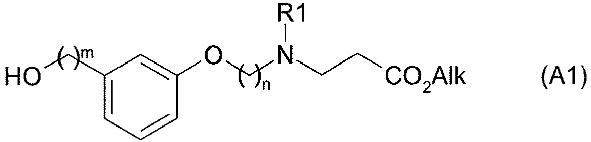 pour laquelle n, m, R1 et Alk sont tels que définis ci-dessus.

### Etape (a1) :

Cette réaction de couplage est avantageusement réalisée en présence d'une base telle que K₂CO₃.
Les produits de départ ((D) et (E)) sont soit commerciaux, soit préparés par des techniques bien connues de l'homme du métier.

### Etape (b1) :

De même, cette étape est avantageusement réalisée en présence d'une base comme K₂CO₃.
Le réactif (G) est facilement synthétisé par des procédés bien connus de l'homme du métier comme illustré dans les exemples ci-dessous.

### DESCRIPTIF DES FIGURES ANNEXEES :

La figure 1 représente le taux de survie de souris traitées, ou non, avec des « molécules simplifiées » 1 et 2 de l'invention, après injection de cellules tumorales, en fonction du nombre de jours après injection de ces cellules.
La figure 2 montre des clichés radiographiques de souris non traitées (contrôle) ou traitées avec la molécule 4 ou le sel disodique d'acide zolédronique, après 4 semaines de traitement.
La figure 3 représente des clichés radiographiques de souris non traitées (contrôle) ou traitées avec la molécule (IV) démontrant un effet inhibiteur de la dégradation osseuse caractérisé par l'augmentation de la densité minérale osseuse en zone épiphysaire (flèche blanche). Cet effet démontre le tropisme de la molécule (IV) pour la fraction minérale osseuse.
La figure 4 représente l'évolution de la taille de la tumeur en fonction du temps avec ou sans traitement à J7 avec la molécule (IV) ou l'ifosfamide (molécule de chimiothérapie de référence). Les losanges représentent le contrôle (c'est-à-dire une absence de traitement), les carrés représentent les résultats obtenus avec l'ifosfamide utilisé à 230 µmoles/kg et les triangles représentent les résultats obtenus avec le composé (IV) utilisé à 115 µmoles/kg.

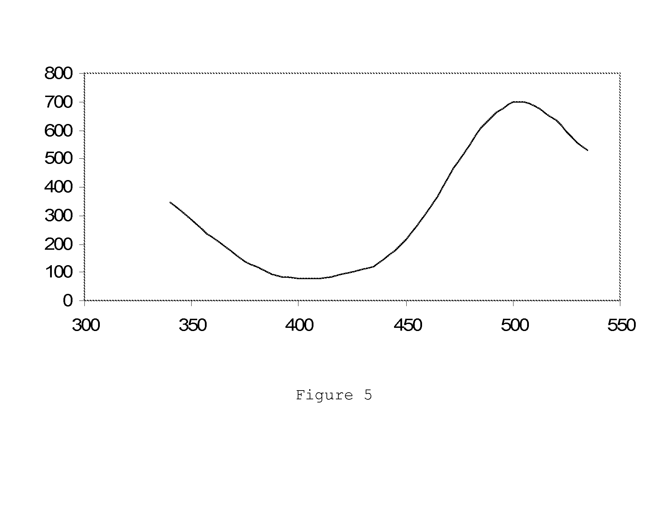

La figure 5 représente le spectre d'émissions de la molécule (II) (exemple comparatif).

### ABREVIATIONS UTILISEES :

| | |
|---|---|
| DCM | Dichlorométhane |
| DMF | Diméthylformamide |
| éq. | Equivalent |
| HMDS | Hexaméthylsilazane |
| HRMS | Spectre de Masse Haute Résolution |
| ppm | partie par million |
| PTSA | Acide *para*-toluènesulfonique |
| RMN | Résonance Magnétique Nucléaire |
| TA | Température Ambiante |
| TEA | Triéthylamine |
| THF | Tétrahydrofurane |

### EXEMPLES :

### 1. Synthèse des molécules

### 1.1. Synthèse de la molécule de formule (II) (exemple comparatif)

La synthèse de la molécule de formule (II) a été réalisée en 8 étapes à partir du 3-hydroxy-benzaldéhyde et du N-méthyl-3-amino-propanoate de *tert*-butyle, selon le schéma réactionnel suivant :

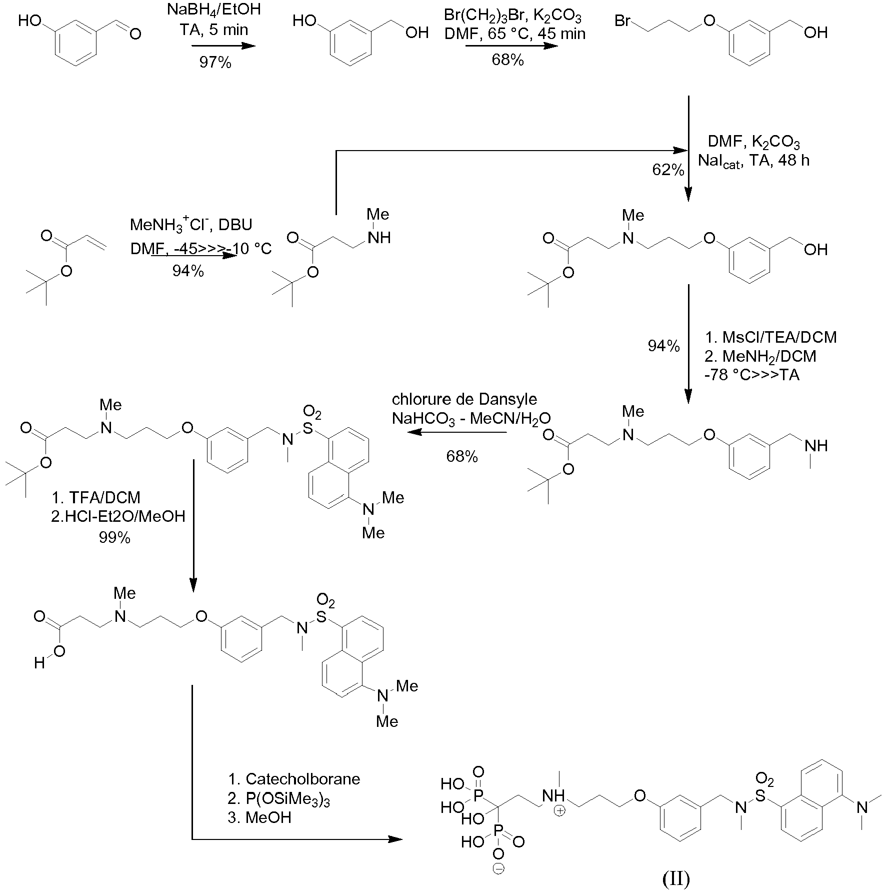

### Alcool 3-(hydroxy)benzylique :

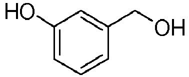

NaBH₄ (775 mg, 20,5 mmol) est ajouté progressivement à une solution de 3-hydroxybenzaldéhyde (5 g, 40,9 mmol) dans EtOH (25 ml) en refroidissant le mélange réactionnel avec de l'eau froide. Ce mélange est agité pendant 5-10 min à la même température. Puis du dichlorométhane (DCM) (100 ml) est ajouté suivi par une solution aqueuse 2M de HCl (jusqu'à pH∼3). La phase organique est séparée et le produit final est extrait 4 fois de la phase aqueuse par un mélange EtOH /DCM en proportion 1:3. Les phases organiques réunies sont séchées sur Na₂SO₄. Après évaporation des solvants sous pression réduite une huile incolore visqueuse est obtenue (4,9 g, 97%), qui cristallise peu à peu.
RMN ¹H (CD₃OD, 300 MHz) δ, ppm: 7,13 (1H, t, ³*J* =9 Hz); 6,90-6,72 (2H, m); 6,71-6,60 (1H, m); 4,83 (2H, s); 4,53 (2H, s).
RMN ¹³C (CD₃OD, 300 MHz) δ, ppm: 158,65; 144,37; 130,49; 119,22; 115,26; 114,90; 65,29.

### (3-(3-Bromopropoxy)phényl)méthanol :

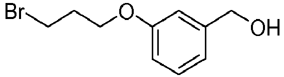

Le 1,3-dibromopropane (23 ml, 0,22 mol) est ajouté progressivement à une suspension de K₂CO₃ (3,2 g, 23 mmol) et de 3-(hydroxyméthyl)phénol (5 g, 40,3 mmol) dans le diméthylformamide (DMF) (30 ml) agitée préalablement pendant 5 min à 65 °C. Le mélange réactionnel est maintenu sous agitation pendant 45 min à la même température. Après refroidissement à température ambiante (TA), Et₂O (150 ml) est ajouté et la phase organique est extraite 4 fois par une solution aqueuse saturée de NaCl. La phase organique est séchée sur Na₂SO₄. Après évaporation du solvant sous pression réduite une huile incolore visqueuse est obtenue (6,7 g, 68%), qui cristallise peu à peu.
RMN ¹H (CDCl₃, 300 MHz) δ, ppm: 7,22 (1H, t, *³J* =9 Hz); 6,94-6,83 (2H, m); 6,82-6,73 (1H, dd, *³J =* 9 Hz, *J* = 3 Hz); 4,58 (2H, s); 4,05 (2H, t, *³J =* 6 Hz); 3,56 (2H, t, *³J* = 6 Hz); 2,46 (1H, br, s) ; 2,27 (2H, q, ³*J* = 6 Hz).
RMN ¹³C (CDCl₃, 300 MHz) δ, ppm: 159,00; 142,74; 129,68; 119,44; 113,82; 113,01; 65,41; 65,05; 32,46; 30,17.

### 3-(Méthylamino)propanoate de tert-butyle :

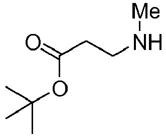

L'acrylate de tert-butyle (3,08 g, 3,52 ml, 24 mmol) est ajouté progressivement à -45 °C à la solution homogène obtenue à partir de DMF (40 ml), de chlorure de méthylammonium (4,9 g, 72 mmol) et de 1,8-diazabicyclo[5.4.0]undec-7-ène (DBU) (21 ml, 144 mmol). Le mélange réactionnel est maintenu sous agitation - 10 °C pendant 2h30. Puis Et₂O (200 ml) est ajouté et le milieu réactionnel est extrait 4 fois par une solution aqueuse saturée de NaCl. La phase organique est séchée sur Na₂SO₄. Après évaporation du solvant sous pression réduite à 30 °C, une huile incolore est obtenue (3,6 g, 94%).
RMN ¹H (CDCl₃, 300 MHz) δ, ppm: 2,75 (2H, t, ³*J* = 6 Hz); 2,42-2,32 (5H, m) ; 1, 40 (9H, s).
RMN ¹³C (CDCl₃, 300 MHz) δ, ppm: 171,97; 80,18; 47,24; 36,16; 35,57; 27,99.

### 3-((3-(3-Hydroxyméthyl)phénoxy)propyl)(méthyl)amino)propanoate de tert-butyle :

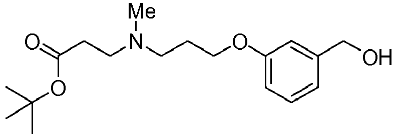

K₂CO₃ (1 g, 7,3 mmol) est ajouté à TA à une solution de (3-(3-bromopropoxy)phényl)méthanol (1,8 g, 7,3 mmol) et de 3-(méthylamino)propanoate de *tert*-butyle (1,75 g, 11 mmol) dans le DMF (50 ml) contenant une quantité catalytique de NaI. Ce mélange est agité pendant 48 h à la même température sous argon. Puis Et₂O (200 ml) est ajouté et cette solution extraite 4 fois par une solution aqueuse saturée de NaCl. La phase organique est séchée avec Na₂SO₄. Après la chromatographie sur silice (éluant - DCM : MeOH = 100 : 1) et évaporation du solvant sous pression réduite à 30 °C, une huile incolore est obtenue (1,46 g, 62%). RMN ¹H (CDCl₃, 300 MHz) δ, ppm: 7,18 (1H, t, ³*J* = 9 Hz); 6,93-6,80 (2H, m); 6,79-6,68 (1H, dd, *³J =* 9 Hz, *J =* 3 Hz); 4,57 (2H, s); 3,92 (2H, t, ³*J* = 6 Hz); 3,22 (1H, br s); 2,62 (2H, t, ³*J* = 6 Hz); 2,45 (2H, t, *³J =* 6 Hz); 2,32 (2H, t, *³J =* 6 Hz); 2,17 (3H, s); 1,86 (2H, q, ³*J* = 6 Hz); 1,39 (9H, s).
RMN ¹³C (CDCl₃, 300 MHz) δ, ppm: 172,12; 159,30; 143,04; 129,52; 119,07; 113,74; 112,91; 80,51; 66,15; 64,95; 54,07; 52,96; 41,98; 33,62; 28,20; 27,17.

### 3-(Méthyl(3-(3-((méthylamino)méthyl)phénoxy)propyl)-amino) propanoate de tert-butyle :

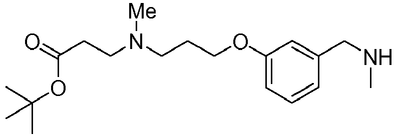

Le chlorure de méthanesulfonyle (MsCl) (0,555 ml, 7.2 mmol) est ajouté progressivement à -78 °C à une solution de 3-((3-(3-(hydroxyméthyl)-phénoxy)propyl) (méthyl)amino)propanoate de *tert*-butyle (465 mg, 1,44 mmol) et de triéthylamine (TEA) (1,4 ml, 10 mmol) dans le dichlorométhane (20 ml). Ce mélange est agité pendant 1h30 à la même température sous argon. Puis la méthylamine (0,65M solution dans le dichlorométhane, 22 ml, 14,3 mmol) est ajoutée, on laisse revenir à température ambiante et agiter à cette température pendant encore 1 h. Le mélange obtenu est extrait 4 fois par une solution aqueuse mixte de NaCl avec NaHCO₃. La phase organique est séchée sur Na₂SO₄. Après évaporation de solvant sous pression réduite le produit final brut est obtenu sous la forme d'une huile incolore (455 mg, 94%), qui est utilisée sans purification supplémentaire pour l'étape suivante.
RMN ¹H (CDCl₃, 300 MHz) δ, ppm: 7,30 (1H, t, *J* = 8 Hz) ; 7,11 (1H, s); 7,02 (1H, d, *J* = 6 Hz); 6,96 (1H, d, *J* = 6 Hz) ; 4,55 (2H, s); 4,07 (2H, t, *J =* 6 Hz); 3,0-2,83 (4H, m); 2,69 (3H, s); 2,60 (2H, t, *J* = 6 Hz); 2,52 (3H, s); 2,10 (2H, q, *J* = 6 Hz); 1,40 (9H, s).
RMN ¹³C DEPT-135 (CDCl₃, 300 MHz) δ, ppm: 130,49; 124,30; 118,92; 116,54; 66,11; 61,06; 53,83; 52,65; 41,52; 39,54; 32,87; 28,06; 26,32.

### 3-((3-(3-(((5-(Diméthylamino)naphthalèn-1-yl)(méthyl)amino) méthyl)phénoxy)propyl)-(méthyl)amino)propanoate de tert-butyle :

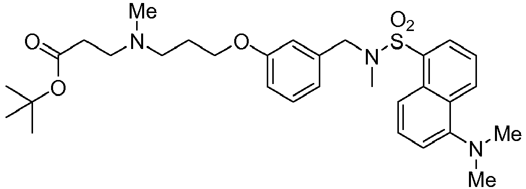

La solution de NaHCO₃ (133 mg, 1,58 mmol) dans l'eau (2 ml) est ajoutée à TA à la solution de 3-(méthyl(3-(3-((méthylamino)méthyl)phénoxy)propyl)amino)propanoate de tert-butyle brut (455 mg, 1,35 mmol) et de chlorure de dansyle (364 mg, 1,35 mmol) dans l'acétonitrile (35 ml). Ce mélange est agité pendant 1h30 à la même température sous argon. Puis le solvant est évaporé sous pression réduite et l'huile obtenue est purifiée par chromatographie sur gel de silice (éluant - DCM suivi par le mélange DCM:MeOH = 100:1). Le produit final est obtenu sous la forme d'une gomme amorphe vert-clair fluorescente (522 mg, 68%).
RMN ¹H (CDCl₃, 300 MHz) δ, ppm: 8,58 (1H, d, *J =* 9 Hz); 8,46 (1H, d, *J* = 9 Hz); 8,26 (1H, d, *J* = 6 Hz); 7,65-7,50 (2H, m); 7,28-7,15 (2H, m); 6,87-6,70 (3H, m); 4,32 (2H, s); 3,87 (2H, t, *J* = 6 Hz); 2,92 (6H, s); 2,80-2,63 (5H, m); 2,55 (2H, t, *J* = 6 Hz); 2,43 (2H, t, *J* = 6 Hz); 2,29 (3H, s); 1,93 (2H, q, *J* = 6 Hz); 1,46 (9H, s).
RMN ¹³C (CDCl₃, 300 MHz) δ, ppm: 172,08; 159,47; 151,97; 137,52; 134,07; 130,72; 130,46; 130,42; 130,27; 129,70; 128,27; 123,37; 120,70; 119,91; 115,36; 114,38; 114,13; 80,55; 66,07; 54,18; 53,65; 53,13; 45,61; 42,08; 33,91; 33,77; 28,28; 27,24.

### Acide 3-((3-(3-((5-(diméthylamino)-N-méthylnaphthalène-1-sulfonamido)méthyl)phénoxy)propyl)(méthyl)amino)propanoïque :

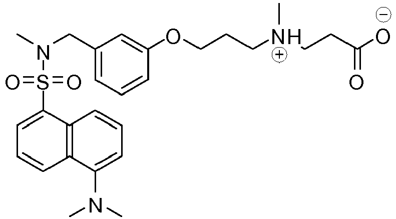

L'acide trifluoroacétique (2 ml) est ajoutée à une solution de 3-((3-(3-(((5-(diméthylamino)naphthalèn-1-yl)(méthyl)amino) méthyl)phénoxy)propyl)(méthyl)amino)propanoate de tert-butyle (100 mg, 0,175 mmol) dans du dichlorométhane (4 ml) à TA. Ce mélange est agité pendant 4,5 h à la même température. Puis le solvant est coévaporé avec du dichlorométhane sous pression réduite et l'huile obtenue est purifiée par chromatographie sur colonne de gel de silice (éluant - DCM suivi par le mélange DCM:MeOH=10:1). Le produit final est obtenu sous la forme d'une gomme amorphe vert-clair fluorescente (90 mg, 99%).
RMN ¹H (CDCl₃-MeOD, 300 MHz) δ, ppm: 8, 60-8, 45 (2H, m); 8,17 (1H, d, *J* = 6 Hz); 7,65-7,50 (2H, m); 7,37 (1H, d, *J* = 6 Hz); 7,12 (1H, t, *J =* 6 Hz); 6,80-6,65 (3H, m); 4,25 (2H, s); 3,87 (2H, t, *J* = 6 Hz) ; 3,35-3,10 (4H, m); 3,00 (6H, s); 2,85-2,70 (5H, m); 2,65 (3H, s) ; 2,14 (2H, m).
RMN ¹³C (CDCl₃-MeOD, 300 MHz) δ, ppm: 172,23; 158,51; 148,22; 137,45; 134,27; 130,28; 130,11; 129,77; 129,47; 129,02; 128,02; 124,32; 121,80; 121,18; 116,38; 114,03; 113,93; 64,46; 54,39; 53,42; 51,81; 45,76; 40,18; 33,95; 28,84; 23,89.

### Acide 3-((3-(3-((5-(diméthylamino)-N-méthylnaphthalène-1-sulfonamido)méthyl)phénoxy)propyl)(méthyl)amino)-1-hydroxypropane-1,1-diyldiphosphonique (II) :

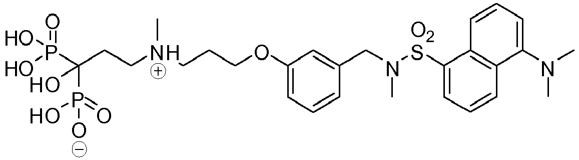

Une solution de catécholborane (1M dans le THF, 1,07 ml, 1,07 mmol, 6,1 éq.) a été ajoutée sur l'acide 3-((3-(3-((5-(diméthylamino)-N-méthylnaphthalène-1-sulfonamido)méthyl) phénoxy)propyl)(méthyl)amino)propanoïque (90 mg, 0,175 mmol, 1 éq.) sous argon à TA à sec. Ce mélange est agité pendant 1 h à la même température jusqu'à la fin du dégagement gazeux (H₂). Puis P(OSiMe₃)₃ (371 mg, 1,24 mmol, 7,1 éq.) a été additionné pur, et l'agitation a été maintenue pendant 16 h. Du méthanol (0,5 ml) est ajouté, le mélange réactionnel est agité pendant 1 h puis dilué avec du DCM (15 ml). Un précipité blanc se forme. Il est filtré, rincé rapidement par du DCM et séché sous argon. On obtient 60 mg d'une poudre blanche fluorescente hygroscopique qui sera purifié sur colonne de gel de silice (Silica gel 100 C18-Reversed phase, éluant H₂O :MeOH). RMN ¹H (D₂O-Py-DMSO-MeOD, 300 MHz) δ, ppm: 8,33 (1H, d, *J* = 6 Hz); 8,17 (1H, d, *J* = 6 Hz); 8,02 (1H, d, *J* = 6 Hz); 7,60-7,40 (2H, m); 7,11 (1H, d, *J* = 6 Hz); 7,05 (1H, t, *J* = 6 Hz) ; 6,73 (1H, d, *J* = 6 Hz); 6,64 (1H, d, *J* = 6 Hz); 6,59 (1H, s); 4,13 (2H, s); 3,79 (2H, t, *J* = 6 Hz); 3,37 (2H, m); 2,78 (3H, s); 2,64 (6H, s); 2,56 (3H, s); 2,55-2,20 (4H, m); 2,08 (2H, m).

RMN ¹³C (D₂O, 300 MHz) δ, ppm: 158,25; 151,26; 137,14; 133,93; 130,06 (sl); 129,74 (sl); 129,38; 128,20 (sl); 126,51 (sl); 123,53 (sl); 120,50 (sl); 119,17 (sl); 115,17 (sl); 114,24 (sl); 113,84 (sl); 72,12 (t, J = 132 Hz); 64,81; 53,50; 53,04; 44,71; 39,29; 38,74; 33,60; 28,07; 23,64.
RMN ³¹P (D₂O-Py-DMSO-MeOD, 300 MHz) δ, ppm: 16.
HRMS (ES) (m/z) : [M+H]⁺ calculé pour C₂₇H₃₉N₃O₁₀P₂S 660,1910, trouvé 660,1911

### 1.2. Synthèse de la molécule de formule (III)

La synthèse est réalisée à partir de la molécule 3 dont le protocole de synthèse est décrit ci-dessous (voir 1.4.).

### Acide 3-((3-(3-((amino(bis(2-chloroéthyl)amino)phosphoryloxy) méthyl)phénoxy)propyl)(méthyl)amino)propanoïque :

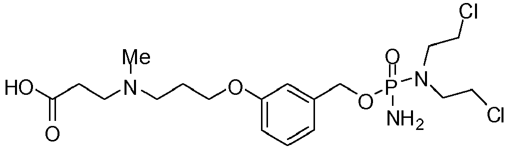

De l'acide trifluoroacétique (TFA) (20 ml) est rajouté à une solution de la **molécule 3** (1,08 g, 2,05 mmol) dans le MeNO₂ (160 ml) et la solution obtenue est agitée 30 min à TA. Le solvant est évaporé 10 min à TA et à 40 °C à sec, puis repris au DCM pour éliminer les traces de TFA. Le résidu est rapidement chromatographié sur colonne de gel de silice (40 cm³, gradient DCM jusqu'à DCM/MeOH (1:1)) pour donner une huile incolore (267 mg, 33%).
RMN ¹H (CD₃OD, 300 MHz) δ, ppm: 7,29 (1H, t, *J =* 7 Hz), 7,08-6,87 (3H, m), 4,96 (2H, d, *J* = 8 Hz), 4,14 (2H, t, *J* = 7 Hz), 3,70-3,55 (4H, m), 3, 50-3, 30 (8H, m), 2,89 (3H, s), 2,59 (2H, t, *J* = 7 Hz), 2,26 (2H, m).
RMN ¹³C (CD₃OD, 300 MHz) δ, ppm: 177,63 ; 160,24 ; 140,12 (d, *J =* 7 Hz) ; 130,88 ; 121,45 ; 115,61 ; 114,86 ; 68,09 (d, *J* = 5 Hz) ; 66,36 ; 55,20 ; 55,04 ; 50,81 (d, *J =* 5 Hz) ; 43,24 ; 40,35 ; 31,22 ; 25,57.
RMN ³¹P (D₂ O, 300 MHz) δ, ppm: 20.
HRMS (ES) (m/z): [M+H]⁺ calculé pour C₁₈H₃₀Cl₂N₃O₅P 470,1378, trouvé : 470,1377.

### Acide 3-((3-(3-((amino(bis(2-chloroéthyl)amino)phosphoryloxy) méthyl)phénoxy)propyl)(méthyl)amino)-1-hydroxypropane-1,1-diyl-diphosphonïque (III) :

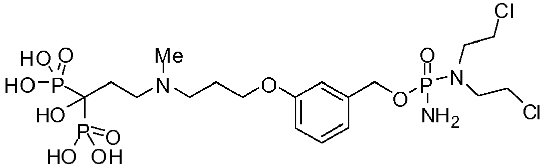

Le même protocole que celui utilisé pour la synthèse de la molécule (II) (dernière étape) est appliqué, en utilisant toutefois une solution de 230 mg d'acide 3-((3-(3-((amino(bis(2-chloroéthyl)amino)phosphoryloxy)méthyl)phénoxy)propyl)(méthyl) amino)propanoïque dans 6,5 ml de THF, 5,1 mol. éq. de catécholborane et 6,1 mol. éq. de P(OSiMe₃)₃. On isole un produit incolore avec un rendement de 32%.
RMN ¹H (D₂O, 300 MHz) δ, ppm: 7,36 (1H, t, *J* = 7 Hz), 7,11-6,92 (3H, m), 4,97 (2H, d, *J* = 8 Hz), 4,15 (2H, t, *J* = 7 Hz), 3,72-3,56 (4H, m), 3,56-3,10 (8H, m), 2,86 (3H, s), 2,33 (2H, m), 2,21 (2H, m).
RMN ¹³C (D₂O, 300 MHz) δ, ppm: 158,16 ; 138,10 (d, *J =* 7 Hz) ; 130,19 ; 120,82 ; 115,00 ; 113,98 ; 72,10 (t, ¹*J*_{C-P} = 130 Hz) ; 67,31 (d, *J* = 5 Hz) ; 65, 35 ; 53,62 ; 53, 29 (t, *J=* 7 Hz) ; 47,93 (d, *J* = 5 Hz) ; 42,06 ; 39,55 ; 27,95 ; 23,67.
RMN ³¹P (D₂O, 300 MHz) δ, ppm: 20 (1P), 18 (2P).
HRMS (ES) (m/z): [M+Na]⁺ calculé pour C₁₈H₃₄Cl₂N₃O₁₀P₃ 638, 0734, trouvé : 638,0733.

### 1.3. Synthèse de la molécule de formule (IV)

### 2-(3-(3-Bromopropoxy)benzyloxy)éthanol :

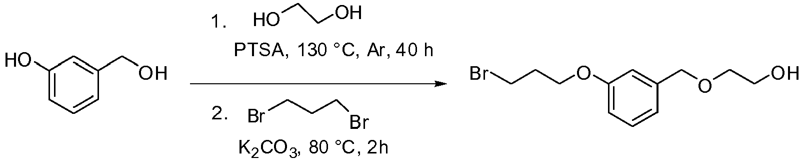

La solution de 3-(hydroxyméthyl)phénol (8,8 g, 71 mmol) et d'acide paratoluènesulfonique (PTSA) (2,3 g, 13,4 mmol) dans l'éthylèneglycol (180 ml) est chauffée à 130 °C sous argon pendant 40 h. Après refroidissement à 80 °C, le K₂CO₃ (13 g, 94 mmol) puis le 1,3-dibromopropane (42 ml, 386 mmol) sont rajoutés. Au bout de 2 h d'agitation à cette température, la reaction est refroidie à TA et extraite avec un mélange DCM-H₂O. Puis la phase organique est lavée plusieurs fois à l'eau, séchée sur Na₂SO₄ et concentrée sous vide. Le produit final brut est obtenu sous la forme d'une huile jaune (14 g, 68% pour deux étapes), et utilisé sans autre purification.
RMN ¹H (CDCl₃, 300 MHz) δ, ppm: 7,28 (1H, t, *J =* 7 Hz), 7,00-6,90 (2H, m), 6, 85 (1H, dd, *J =* 3 Hz, *J* = 7 Hz), 4, 54 (2H, s), 4, 11 (2H, t, *J* = 7 Hz), 3,77 (2H, t, *J* = 6 Hz), 3, 68-3, 52 (4H, m), 2,62 (1H, br s), 2,32 (2H, q, *J =* 7 Hz).
RMN ¹³C (CDCl₃, 300 MHz) δ, ppm: 158,97; 139,73; 129,66; 120,36; 113,95; 113,92; 73,20; 71,56; 65,35; 61,91; 32,46; 30,21.

### 3-((3-(3-((2-Hydroxyéthoxy)méthyl)phénoxy)propyl)(méthyl)amino) propanoate de tert-butyle.

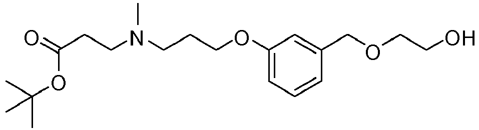

Le même protocole que celui décrit pour la préparation du 3-((3-(3-(hydroxyméthyl)phénoxy)propyl)(méthyl)amino)propanoate de *tert*-butyle est appliqué. On isole un produit incolore avec un rendement de 68%.
RMN ¹H (CDCl₃, 300 MHz) δ, ppm: 7,20 (1H, t, *J =* 7 Hz), 6,90-6,81 (2H, m), 6,78 (1H, dd, *J* = 3 Hz, *J* = 7 Hz), 4, 48 (2H, s), 3, 96 (2H, t, *J* = 7 Hz), 3,71 (2H, t, *J* = 6 Hz), 3,54 (2H, t, *J =* 6 Hz), 2,65 (2H, t, *J* = 7 Hz), 2,59 (1H, br s), 2,49 (2H, t, *J* = 7 Hz), 2,35 (2H, t, *J* = 7 Hz), 2,21 (3H, s), 1,90 (2H, q, *J* = 7 Hz), 1,40 (9H, s).
RMN ¹³C (CDCl₃, 300 MHz) δ, ppm: 172,13; 159,32; 139,69; 129,54; 119,97; 113,97; 113,87; 80,46; 73,26; 71,56; 66,17; 61,93; 54,11; 53,10; 42,08; 33,78; 28,23; 27,28.

### N-(3-{3-[6-amino-9-chloro-7-(2-chloroéthyl)-6-oxido-2,5-dioxa-7-aza-6-phosphanon-1-yl]phénoxy}propyl)-N-méthyl-β-alaninate de tert-butyle.

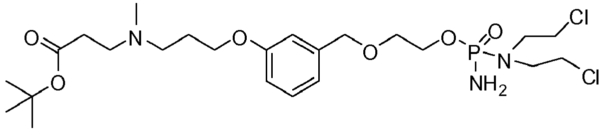

Le même protocole que celui décrit pour la préparation de la **molécule 3** est appliqué. On isole un produit incolore avec un rendement de 65%.
RMN ¹H (CDCl₃, 300 MHz) δ, ppm: 7,20 (1H, t, *J* = 7 Hz), 6,91-6,81 (2H, m), 6, 78 (1H, dd, *J* = 3 Hz, *J* = 7 Hz), 4,48 (2H, s), 4, 19 (1H, m), 4,02 (1H, m), 3,95 (2H, t, *J* = 7 Hz), 3,70-3,50 (6H, m), 3, 97-3, 28 (4H, m), 3,06 (2H, br d, *J =* 3 Hz), 2,65 (2H, t, *J* = 7 Hz), 2,50 (2H, t, *J* = 7 Hz), 2,35 (2H, t, *J* = 7 Hz), 2,21 (3H, s), 1,90 (2H, q, *J=* 7 Hz), 1,39 (9H, s).
RMN ¹³C (CDCl₃, 300 MHz) δ, ppm: 172,07; 159,34; 139,23; 129,65; 120,05; 114,05; 114,02; 80,48; 73,28; 69,53 (d, *J* = 6 Hz); 66,18; 64,75 (d, *J* = 4 Hz); 54,11; 53,11; 49,62; 49,57; 42,68; 42,05; 33,77; 28,23; 27,28.

### N-(3-{3-[6-Amino-9-chloro-7-(2-chloroéthyl)-6-oxido-2,5-dioxa-7-aza-6-phosphanon-1-yl]phénoxy}propyl)-N-méthyl-β-alanine :

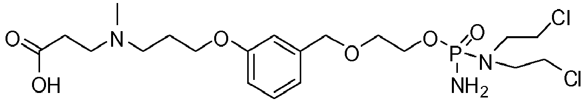

Le même protocole que celui décrit pour la préparation de l'acide 3-((3-(3-((amino(bis(2-chloroéthyl)amino)phosphoryloxy) méthyl)phénoxy)propyl)(méthyl)amino)propanoïque est appliqué. On isole un produit incolore avec un rendement de 73%.
RMN ¹H (CD₃OD, 300 MHz) δ, ppm: 7,26 (1H, t, *J* = 7 Hz), 7,02-6,92 (2H, m), 6,89 (1H, dd, *J* = 3 Hz, *J* = 7 Hz), 4,54 (2H, s), 4,60-4,02 (4H, m), 3, 77-3, 56 (6H, m), 3, 50-3, 30 (8H, m), 2,89 (3H, s), 2,59 (2H, t, *J* = 7 Hz), 2,25 (2H, m).
RMN ¹³C (CD₃OD, 300 MHz) δ, ppm: 177,58; 160,22; 141,26; 130,73; 121,80; 115,18; 115,10; 74,12; 70,73 (d, *J* = 7 Hz); 66,27; 66,03 (d, *J =* 4 Hz); 55,18; 54,99; 50,88; 43,28; 40,40; 31,26; 25,59.

### Acide {3-[(3-{3-[6-amino-9-chloro-7-(2-chloroéthyl)-6-oxido-2,5-dioxa-7-aza-6-phosphanon-1-yl]phénoxy}propyl)(méthyl)amino]-1-hydroxypropane-1,1-diyl}bisphosphonique (IV) :

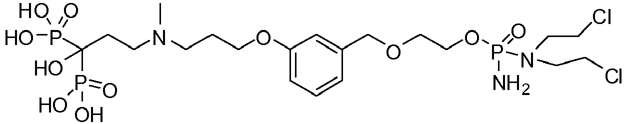

Le même protocole que celui utilisé pour la préparation de l'acide {3-((3-(3-((amino(bis(2-chloroéthyl)amino)phosphoryloxy) méthyl)phénoxy)propyl)(méthyl)amino)-1-hydroxypropane-1,1-diyl} bisphosphonique est appliqué. On isole un produit incolore avec un rendement de 47%.
RMN ¹H (D₂O, 300 MHz) δ, ppm: 7,38 (1H, t, *J* = 7 Hz), 7,13-6,91 (3H, m), 4,59 (2H, s), 4,30-4,00 (4H, m), 3,78 (2H, m), 3,72-3,60 (4H, m), 3,60-3,20 (8H, m), 2,88 (3H, s), 2,35 (2H, m), 2,24 (2H, m).
RMN ¹³C (D₂O, 300 MHz) δ, ppm: 158,13; 139,11; 130,07; 121,47; 114,63; 72, 58; 72,11 (t, ¹*J*_{C-P}= 130 Hz); 69, 00 (d, *J* = 7 Hz); 65,34; 64,84 (d, *J* **=** 4 Hz); 53,65; 53,32 (t, *J* = 6 Hz) ; 47,97; 47,92; 42,08; 39,56; 27,96; 23,71.
RMN ³¹P (D₂O, 300 MHz) δ, ppm: 20 (1P), 18 (2P).
HRMS (ES) (m/z): [M-H]⁻ calculé pour C₂₀H₃₈Cl₂N₃O₁₁P₃ 658,1018, trouvé : 658.1018.

### 1.4. Synthèse de molécules simplifiées de l' invention (exemples comparatifs)

### Molécule 1 :

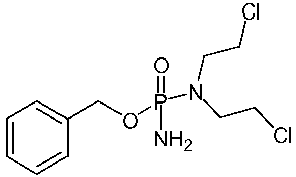

Dans un ballon refroidi par un bain de glace (0°C), équipé d'un réfrigérant et d'une agitation magnétique, sous atmosphère inerte, 484 mg d'hexaméthyldisilazane (3 mmol, 1éq.) sont mis en solution dans 10 mL de THF anhydre. 10 min après addition à 0°C de 2,0 mL de nBuLi (1,6 M dans l'hexane), 1,2 éq. de cette solution est transférée par canulation dans un ballon contenant 324 mg d'alcool benzylique (3 mmol, 1éq.) en solution dans 10 ml de THF anhydre. Après 10 min à 0°C, l'alcoolate ainsi formé est transféré par canulation dans un ballon contenant 777 mg de dichloro-N,N-bis-(2-chloroéthyl)phosphoramide (3mmol, 1éq.) dissout dans 20 mL de THF anhydre. On laisse l'agitation 2 h à 0°C. Après refroidissement à -78°C, le milieu réactionnel est soumis à un bullage d'ammoniac gazeux pendant 30 min. Un dégazage de l'ammoniac dissout dans la solution est effectué pendant 30 min à TA avant évaporation sous pression réduite du solvant. Le produit est directement purifié par chromatographie sur colonne de gel de silice (éluant : CH₂Cl₂/MeOH = 98/2). Une huile transparente cristallisant à froid (congélateur) est ainsi obtenue (312 mg, rendement = 34 %). Le produit est recristallisé dans l'éther isopropylique.
RMN ¹H (CDCl₃) δ (ppm) : 7,34 (s, 5H); 5,08-4,97 (m, 2H); 3,67-3,56 (m, 4H); 3,49-3,35 (m, 4H); 2,89 (s, 2H, NH₂).

### Molécule 2:

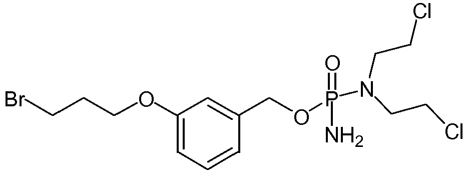

Cette molécule a été préparée selon le protocole de synthèse décrit pour la molécule 3, à partir de l'alcool 3-(3-bromopropoxy)benzylique. Après purification par chromatographie sur colonne de gel de silice (éluant ; DCM/MeOH 99:1), on isole le produit désiré avec un rendement de 66%.
Rf = 0,15 (éluant CH₂Cl₂/MeOH 99:1)
RMN ¹H (CDCl3) δ (ppm) : 7,29 (t, *J* = 8,4 Hz, 1H); 6,96 (d, *J* = 8,4 Hz, 1H); 6,94 (s, 1H) ; 6,87 (d, *J* = 8,4 Hz, 1H) ; 5,07-4,93 (m, 2H); 4,12 (t, *J* = 6 Hz, 2H); 3,65-3,59 (m, 6H); 3,48-3,39 (m, 4H); 2,71 (s, 2H); 2,32 (m, *J* = 6 Hz, 2H).
RMN ³¹P (CDCl3) δ (ppm) : 15,85 (s).

### Molécule 3:

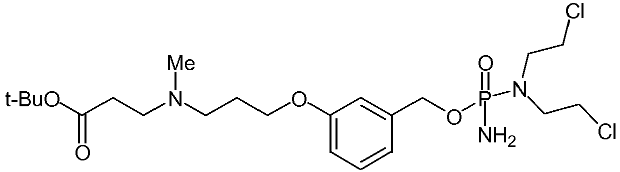

Une solution de NaHMDS (2M dans le THF, 1,12 ml, 2,22 mmol) est ajoutée à -78 °C sous argon à une solution de tert-butyl-3-((3-(3-(hydroxyméthyl)phénoxy)propyl)(methyl)-amino)propanoate (600 mg, 1,86 mmol) dans le THF anhydre (15 mL). Après 5 min d'agitation à -78°C, le mélange réactionnel est agité à TA pendant 15 min et refroidi de nouveau jusqu'à -78 °C. A cette température le dichloro-N,N-bis(2-chloroéthyl)phosphoramide (480 mg, 1.86 mmol) en solution dans le THF anhydre (3 mL) est ajouté au goutte à goutte et le mélange réactionnel est agité pendant 4 h en laissant la température remonter doucement jusqu'à température ambiante, ce qui laisse apparaître un insoluble. Le mélange réactionnel est de nouveau refroidi jusqu'à -50 °C et une solution d'ammoniac (1M dans le DCM, 6 ml, 6 mmol) est ajoutée. Après agitation à TA pendant 2 h, du DCM (120 ml) est ajouté et le solvant est évaporé sous pression réduite. Après chromatographie sur gel de silice (éluant : DCM puis DCM : MeOH = 5 : 1) on obtient le produit attendu sous forme d'une huile incolore (589 mg, rendement = 60%).
RMN ¹H (CDCl₃, 300 MHz) δ, ppm: 7,23 (1H, t, *J* = 8 Hz) ; 6,94-6,77 (2H, m); 5,06-4,82 (2H, m); 3,97 (2H, t, *J* = 7 Hz); 3,65-3,50 (4H, m); 3,96-3,27 (4H, m); 2,87 (2H, br s); 2,65 (2H, t, *J =* 7 Hz); 2,49 (2H, t, *J* = 7 Hz); 2,35 (2H, t, *J* = 7 Hz); 2,21 (3H, s); 1,90 (2H, q, *J* = 7 Hz); 1,40 (9H, s).
RMN ¹³C (CDCl₃, 300 MHz) δ, ppm: 172,16; 159,40; 138,09 (d, ³J_{C-P} = 7 Hz) ; 129,84; 119,97; 114,61; 113,95; 80,51; 67,20 (d, ²J_{C-P} = 5 Hz); 66, 26; 54,10; 53,15; 49, 40 (d, ²J_{C-P} = 5 Hz) ; 42, 73; 42, 13; 33,85; 28,27; 27,31.
RMN ³¹P (CDCl₃, 300 MHz) δ, ppm: 16.

### Molécule 4 :

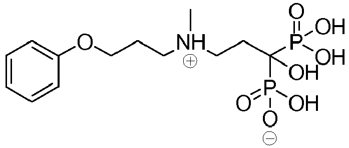

Une solution de catécholborane (1M dans le THF, 2,1 éq.) est ajoutée sur l'acide 3-[méthyl-(3-phénoxypropyl)amino] propanoïque (1 éq.) sous argon à TA. Ce mélange est agité pendant 1 h à la même température jusqu'à la fin du dégagement gazeux (H₂). Puis P(OSiMe₃)₃ (3,1 éq.) est additionné pur, et l'agitation est maintenue pendant 16 h. Du méthanol (2 ml) est ajouté et le mélange réactionnel est agité pendant 1 h, évaporé à sec sous vide, dissout dans un minimum de méthanol et dilué avec de l'éther diéthylique (30 ml). Un précipité blanc se forme. Celui-ci est séparé, rincé rapidement avec de l'éther diéthylique et séché sous argon. Une poudre blanche est ainsi obtenue avec un rendement de 85%.
RMN ¹H (DMSO-d₆, 300 MHz) δ, ppm: 7,30-7,15 (2H, m); 7,23 (2H, t, *J* = 7 Hz) ; 6,97-6,80 (3H, m); 3,99 (2H, t, *J=* 6 Hz); 3,55-3,00 (4H, m); 2,74 (3H, s); 2,35-1,95 (4H, m).
RMN ¹³C (CD₃COCD₃, 300 MHz) δ, ppm: 158,87; 130,16; 121,64; 115,18; 72,33 (t, ¹*J_{C-P}* = 136 Hz); 65,51; 54,31; 53,37; 40,37; 28,28; 24,44.
RMN ³¹P (DMSO-d₆, 300 MHz) δ, ppm: 19.

### 2. Tests biologiques

### 2.1. Molécules à activité anti-tumorale

▪ Les trois molécules suivantes, illustrant des « molécules simplifiées » de l'invention (exemples comparatifs) ont été testées pour leur activité anti-tumorale :

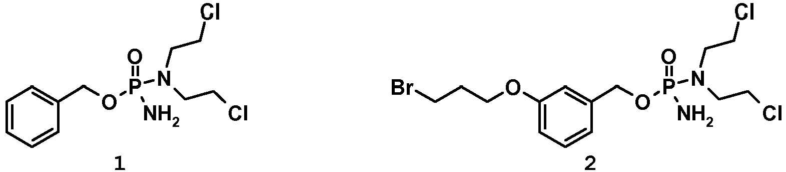

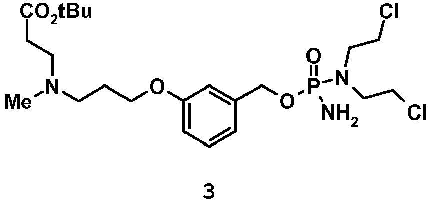

### Test in vitro :

Une étude a été réalisée en parallèle sur une lignée de cellules tumorales (ostéosarcome de souris POS-1) et une lignée de cellules non tumorales à fort taux de prolifération (fibroblastes murins L929). Les cellules ont été cultivées en plaques 96 puits pendant 72 heures en présence des molécules à tester 1 à 3 et en utilisant le DMSO ou l'éthanol comme solvant (1% final dans le puits).

Le tableau suivant présente les résultats obtenus (valeurs des IC50 mesurées) pour les différentes molécules et lignées de cellules.

| | **L929 (DMSO)** | **POS-1 (DMSO)** | **L929 (éthanol)** | **POS-1 (éthanol)** |
|---|---|---|---|---|
| **Molécule 1** | 500 µM | > 500 µM | > 500 µM | > 500 µM |
| **Molécule 2** | >> 500 µM | 200 µM | 250 µM | 300 µM |
| **Molécule 3** | 500 µM | 280 µM | > 500 µM | 500 µM |

Ces résultats montrent bien l'activité inhibitrice de la prolifération cellulaire des molécules 2 et 3 par la partie alkylante phosphorodiamidique de la molécule.

### Test in vivo :

Des souris mâles de souche C3H/HeN âgées de 5 semaines reçoivent chacune 150 000 cellules d'ostéosarcome de souris POS-1 par voie intra-veineuse dans le sinus rétro-orbitaire, induisant le développement de nodules pulmonaires en 4-6 semaines.

Ces souris ont alors été traitées avec une injection de molécules 1 ou 2 (112 µmol/kg), 7 jours après l'injection des cellules tumorales d'ostéosarcome.

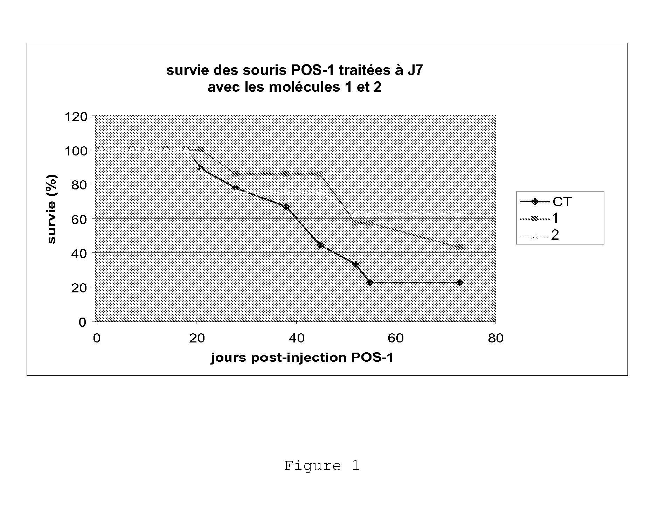

Les résultats des taux de survie, présentés sur la figure 1, montrent une augmentation significative de ce taux de survie dès le 40^{ème} jour post-injection des cellules tumorales chez les souris traitées avec les molécules 1 et 2 (courbe avec des carrés et des triangles, respectivement, sur la figure 1), comparativement aux souris contrôles qui n'ont reçues aucun traitement (courbe CT avec des losanges sur la figure 1). Les molécules 1 et 2 présentent donc bien une activité anti-tumorale dans ce modèle de nodules pulmonaires.

Ces deux tests montrent, de plus, que le résidu R2, qui est ici un principe actif alkylant de type phosphorodiamidique, a été relargué car sinon il n'aurait pu jouer son rôle d'agent alkylant et donc d'agent anti-tumoral. Cela montre donc que lorsque m = 1 et X = O, la molécule est moins stable et il est alors possible de relarguer le principe actif.
▪ Les molécules (III) et (IV) selon l'invention ont été testées également pour leur activité anti-tumorale.

### Test in vitro :

Les activités biologiques (test de viabilité/cytotoxicité) des molécules (III) et (IV) ont été testées *in vitro* sur des cultures de lignées d'ostéosarcome humaines, de souris et de rat en comparaison de cellules normales (ostéoblastes sains issus de calvaria de souris).

Les résultats obtenus *in vitro* démontrent une activité inhibitrice de la prolifération des composés testés dues à une induction de la mort et donc à une activité cytotoxique des composés. Aucune activité cytotoxique n'a été observée sur des cellules ostéoblastiques saines issues de culture primaire de calvaria de souris, démontrant ainsi la non réactivité des ostéoblastes normaux vis-à-vis de ces composés.

Le tableau suivant présente les résultats obtenus (valeurs des IC50 mesurées) pour trois lignées cellulaires d'ostéosarcome humain (MG63), de souris (POS1) et de rat (OSRGA)

| | POS1 | OSRGA | MG63 |
|---|---|---|---|
| Molécule (III) | > 200 µM | > 200µM | > 200µM |
| Molécule (IV) | > 500 µM | > 500 µM | > 500 µM |

### Tests in vivo :

Des cellules POS1 d'ostéosarcome de souris ont été injectées dans le coussinet d'une souris mâle de souche C3H/HeN âgée de 5 semaines. Des fragments de la tumeur qui se développe sur ce site sont ensuite transplantés en site para-osseux afin d'induire le développement d'une tumeur osseuse primitive ostéolytique. La molécule (IV) a alors été injectée à raison d'une cure de 3 injections intraparentérale (IP) de 11,5 ou 115 µmoles/kg à 24 heures d'intervalle à partir du 7^{ème} jour (J7) post-induction tumorale.

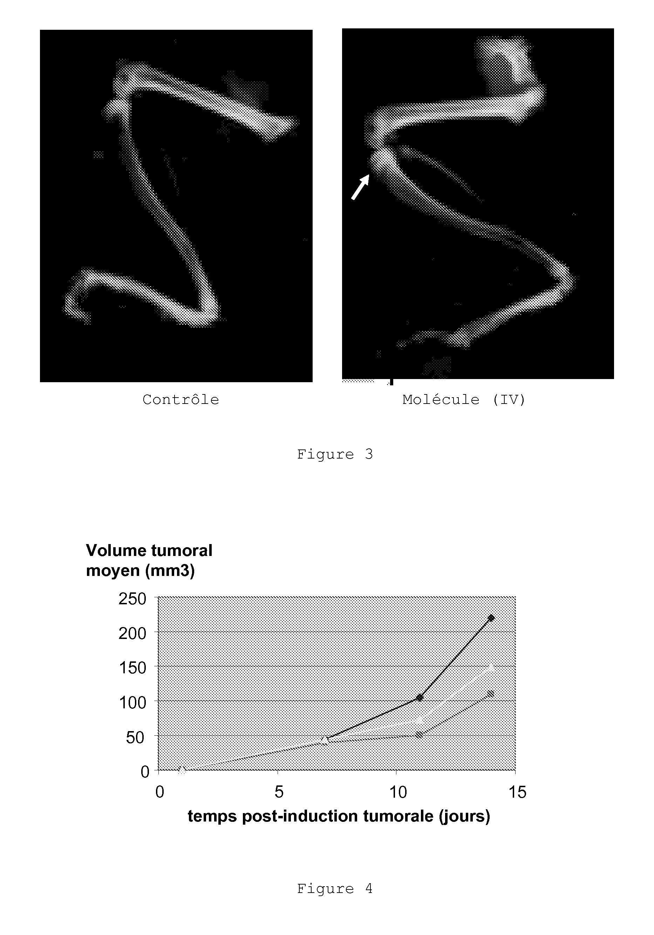

Les résultats obtenus démontrent que le composé complet injecté s'est bien fixé au tissu osseux en augmentant l'apposition osseuse au regard des images radiographiques (voir figure 3) et simultanément a induit une réduction du volume tumoral (site extra-osseux) en libérant le domaine anti-tumoral (voir figure 4), des résultats similaires étant obtenus pour les deux concentrations (-20/-30% de réduction du volume tumoral).

### 2.2. Molécules à fort tropisme osseux

Cette étude a été réalisée en utilisant la molécule 4 suivante, illustrant une « molécule simplifiée » de l'invention :

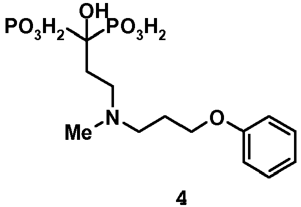

### Test in vivo :

Des souriceaux de 2-3 semaines (souche Swiss) ont été traités avec la molécule 4 ou avec un sel disodique de l'acide zolédronique (dilués dans du PBS) par voie sous-cutanée 2 fois par semaine, à la concentration de 0,35 µmol/kg.

Le sel disodique de l'acide zolédronique est un bisphosphonate de troisième génération qui sert ici de molécule de comparaison.

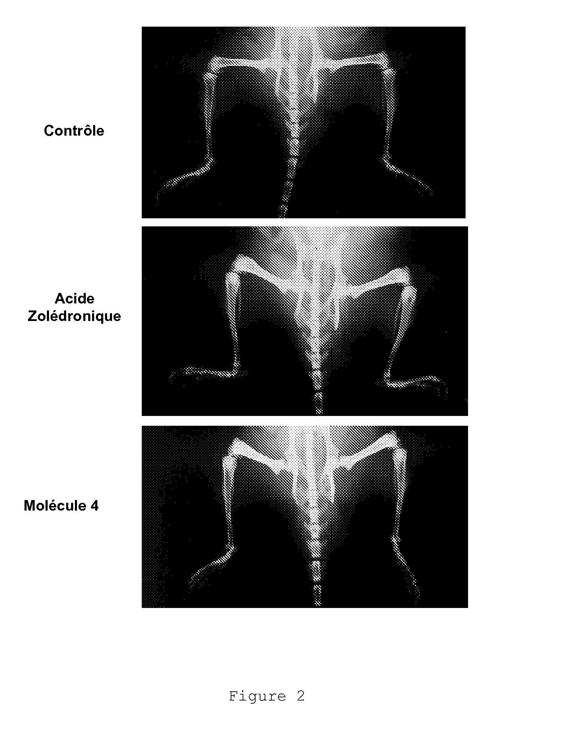

Après 4 semaines de traitement, on constate qu'il n'y a pas d'incidence sur le suivi pondéral, ni sur l'état général des souris traitées avec l'une de ces deux molécules. Les clichés radiographiques présentés sur la figure 2 révèlent un accroissement de la densité minérale après 4 semaines de traitement avec le sel disodique d'acide zolédronique ou avec la molécule 4 par comparaison avec les souris « contrôles ». De plus, le volume osseux spécifique mesuré (BV/TV = volume de l'os rapporté au volume total de tissu analysé) est présenté sur le tableau suivant dans le cas de l'administration de la molécule 4 ou du sel disodique de l'acide zolédronique selon le protocole décrit ci-dessus ou dans le cas d'une absence de traitement (valeur de contrôle).

| | **fémur** | **BV/TV** | **moyenne** |
|---|---|---|---|
| **Contrôle** | gauche | 56,58 % | 62,89 % |
| | droit | 69,20 % | |
| **Sel disodique d'acide zolédronique** | gauche | 72,72 % | 74,02 % |
| | droit | 75,33 % | |
| **Molécule 4** | gauche | 73,69 % | 73,88 % |
| | droit | 74,08 % | |

Ces résultats montrent que la molécule 4 est capable d'induire une augmentation du volume osseux spécifique total des fémurs chez les souris traitées avec cette molécule, comparable aux valeurs obtenues avec le sel disodique de l'acide zolédronique, produit de référence.

### 2.3. Molécules fluorescentes (exemple comparatif)

La molécule (II) a été testée *in vitro* sur différentes lignées cellulaires et ne présente aucune activité cytotoxique. L'administration chez la souris n'a pas non plus révélé d'activité cytotoxique.

La molécule (II) possède la propriété d'absorber de l'énergie lumineuse (λ excitation à 280 nm) et de la restituer rapidement sous forme de lumière fluorescente. Ainsi, le spectre d'émission de la molécule (II), après excitation à 280 nm, révèle que cette molécule ré-émet de la lumière à une longueur d'onde de 500 nm.

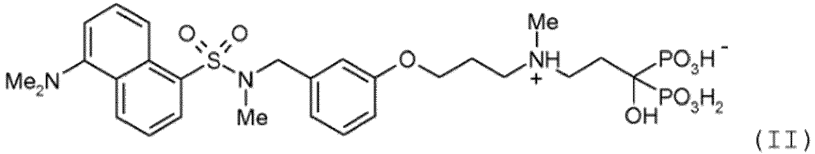

## Revendications

1. Dérivé d'acide hydroxy-bisphosphonique répondant à la formule générale (III) ou (IV) suivante : 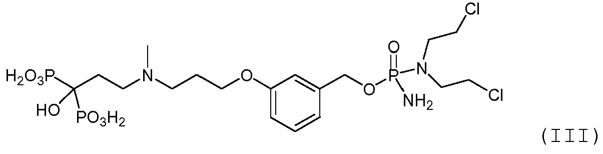 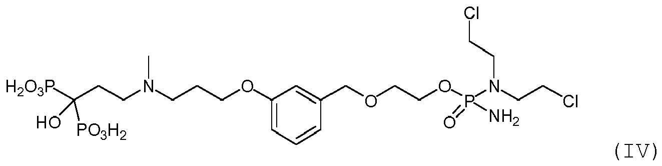 ou un sel pharmaceutiquement acceptable de celui-ci.

2. Dérivé d'acide hydroxy-bisphosphonique selon la revendication 1 ou un sel pharmaceutiquement acceptable de celui-ci pour son utilisation en tant que médicament.

3. Dérivé d'acide hydroxy-bisphosphonique selon la revendication 1 ou un sel pharmaceutiquement acceptable de celui-ci pour son utilisation en tant que médicament pour le traitement d'une pathologie du remodelage osseux ostéolytique ou ostéocondensante choisie parmi les tumeurs osseuses primitives, telles qu'un ostéosarcome, un chondrosarcome, une tumeur à cellules géantes ou un sarcome d'Ewing ; les métastases osseuses ; et le myélome multiple.

4. Composition pharmaceutique comprenant au moins un dérivé d'acide hydroxy-bisphosphonique selon la revendication 1 ou un sel pharmaceutiquement acceptable de celui-ci et au moins un véhicule pharmaceutiquement acceptable.

5. Composition pharmaceutique selon la revendication 4, **caractérisée en ce qu'**elle se présente sous forme d'une solution injectable ou d'un patch.

6. Composition pharmaceutique selon la revendication 4 ou 5 pour son utilisation en tant que médicament.

7. Composition pharmaceutique selon la revendication 6, **caractérisé en ce qu'**elle est utile pour le traitement d'une pathologie du remodelage osseux ostéolytique ou ostéocondensante, telle que les tumeurs osseuses primitives, telles qu'un ostéosarcome, un chondrosarcome, une tumeur à cellules géantes ou un sarcome d'Ewing ; les métastases osseuses ; et le myélome multiple.

8. Composition pharmaceutique selon la revendication 6 ou 7, **caractérisée en ce qu'**elle est formulée pour permettre son administration par voie sous-cutanée, intra-veineuse, per os, intramusculaire ou transdermique.

## Patentansprüche

1. Hydroxy-Biphosphonsäurederivat, das der folgenden allgemeinen Formel (III) oder (IV): 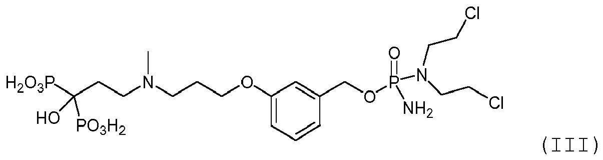 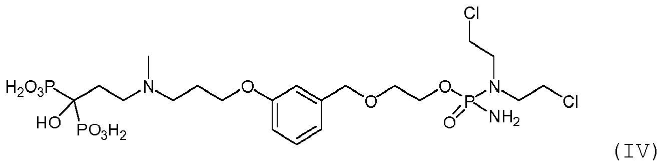 oder einem pharmazeutisch akzeptablen Salz desselben entspricht.

2. Hydroxy-Biphosphonsäurederivat nach Anspruch 1 oder ein pharmazeutisch akzeptables Salz desselben für seine Verwendung als Arzneimittel.

3. Hydroxy-Biphosphonsäurederivat nach Anspruch 1 oder ein pharmazeutisch akzeptables Salz desselben für seine Verwendung als Arzneimittel für die Behandlung einer Pathologie des osteolytischen oder knochenkondensatorischen Knochenremodelings, ausgewählt aus den Knochen-Ersttumoren wie Osteosarkom, Chondrosarcom, Riesenzellentumor oder Ewing-Sarcom, den Knochenmetastasen und dem multiplen Myelom.

4. Pharmazeutische Zusammensetzung, die mindestens ein Hydroxy-Biphosphonsäurederivat nach Anspruch 1 oder ein pharmazeutisch akzeptables Salz desselben und mindestens ein pharmazeutisch akzeptables Vehikel umfasst.

5. Pharmazeutische Zusammensetzung nach Anspruch 4, **dadurch gekennzeichnet, dass** sie in Form einer injizierbaren Lösung oder eines Patchs vorliegt.

6. Pharmazeutische Zusammensetzung nach Anspruch 4 oder 5 für ihre Verwendung als Arzneimittel.

7. Pharmazeutische Zusammensetzung nach Anspruch 6, **dadurch gekennzeichnet, dass** sie für die Behandlung einer Pathologie des osteolytischen oder knochenkondensatorischen Knochenremodelings, wie der Knochen-Ersttumore, wie Osteosarkom, Chondrosarcom, Riesenzellentumor oder Ewing-Sarcom, der Knochenmetastasen und des multiplen Myeloms nützlich ist.

8. Pharmazeutische Zusammensetzung nach Anspruch 6 oder 7, **dadurch gekennzeichnet, dass** sie formuliert ist, um ihre subkutane, intravenöse, per os, intramuskuläre oder transdermale Verabreichung zu erlauben.

## Claims

1. A hydroxy-bisphosphonic acid derivative of the following general formula (III) or (IV): 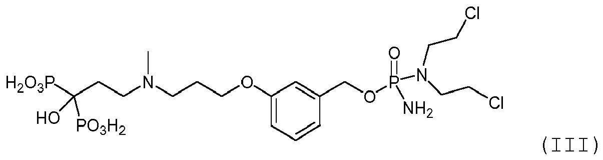 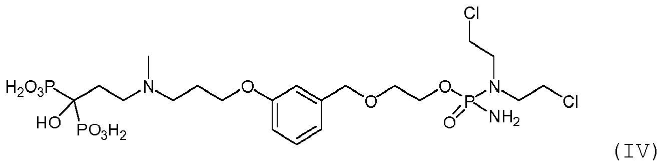 or a pharmaceutically acceptable salt thereof.

2. The hydroxy-bisphosphonic acid derivative according to claim 1 or a pharmaceutically acceptable salt thereof for use as a drug.

3. The hydroxy-bisphosphonic acid derivative according to claim 1 or a pharmaceutically acceptable salt thereof for use as a drug for the treatment of a pathology of osteolytic or osteocondensing bone remodeling selected from primitive bone tumors, such as an osteosarcoma, a chondrosarcoma, a giant cell tumor or Ewing's sarcoma; bone metastases; and multiple myeloma.

4. A pharmaceutical composition comprising at least one hydroxy-bisphosphonic acid derivative according to claim 1 or a pharmaceutically acceptable salt thereof and at least one pharmaceutically acceptable carrier.

5. The pharmaceutical composition according to claim 4, **characterized in that** it appears as an injectable solution or as a patch.

6. The pharmaceutical composition according to claim 4 or 5 for use as a drug.

7. The pharmaceutical composition according to claim 6, **characterized in that** it is useful for the treatment of a pathology of osteolytic or osteocondensing bone remodeling, such as primitive bone tumors, such as an osteosarcoma, a chondrosarcoma, a giant cell tumor or Ewing's sarcoma; bone metastases; and multiple myeloma.

8. The pharmaceutical composition according to claim 6 or 7, **characterized in that** it is formulated so as to allow its administration via a sub-cutaneous, intravenous, oral, intramuscular or transdermal route.
